# EUROPÄISCHE PATENTANMELDUNG

(11) **EP 1 532 931 A1**
(43) Veröffentlichungstag der Anmeldung: **25.05.2005**
(21) Anmeldenummer: 04022580.7
(22) Anmeldetag: 22.09.2004
(51) Int. Cl.: A61B 17/88

(54) **Schraubendreher mit flexiblem Schaft für Knochenschrauben**

(30) Priorität: 24.11.2003 DE 20318263 U; 25.11.2003 DE 20318703 U
(71) Anmelder: Stryker Trauma GmbH, 24232 Schönkirchen (DE)
(72) Erfinder: Prager, Ronald, 24796 Bovenau (DE); Völzow, Stefan, 24248 Mönckeberg (DE)
(74) Vertreter: Patentanwälte Hauck, Graalfs, Wehnert, Döring, Siemons, Schildberg

(57) **Zusammenfassung**

Schraubendreher für Knochenschrauben, insbesondere für Kompressionsund Verriegelungsschrauben, mit
- einem Handgriff,
- einem zentral aus dem Handgriff vorstehenden Schaft, der an seinem Ende ein Schraubendrehwerkzeug aufweist,
- wobei der Schaft einen flexiblen Schaftabschnitt besitzt,
dadurch gekennzeichnet, daß
- der Schaft zwei starre, massive Abschnitte auf beiden Seiten des flexiblen Abschnitts besitzt,
- der flexible Schaftabschnitt ein flexibles Element zur Übertragung eines Drehmoments aufweist und
- mindestens der flexible Schaftabschnitt von einem elastischen Schutzschlauch umgeben ist.

## Beschreibung

Die vorliegende Erfindung betrifft einen Schraubendreher für Knochenschrauben, insbesondere für Kompressions- und Verriegelungsschrauben an einem Knochennagel.

Aus DD 238 724 A1 ist ein medizinischer Schraubendreher bekannt, der einen Schraubenkopf form- und kraftschlüssig umfaßt.

Aus DE 101 18 570 A1 ist ein chirurgischer Bohrer bekannt, dessen Schaft eine zentrale Längsbohrung für einen faseroptischen Positionsgeber aufweist. Der Schaft des Bohrers besitzt eine Kupplung, auf die ein Bohrkopf aufgesetzt werden kann, einen direkt daran anschließenden flexiblen Schaftabschnitt aus gewickeltem Federstahl und einen sich daran anschließenden starren Schaftabschnitt. Durch den Schaft mit starrem und flexiblem Schaftabschnitt erstreckt sich eine durchgehende Längsbohrung, in die ein Positionsgeber eingeführt wird. Ferner wird in DE 101 18 570 A1 vorgeschlagen, einen Schraubendreher oder ein anderes Werkzeug in die Kupplung einzusetzen. Als nachteilig an dem bekannten Bohrer hat sich herausgestellt, daß dieser, insbesondere bei seinem Einsatz als Schraubendreher, nur schlecht zu handhaben ist und eine Reinigung nach dem Gebrauch einen beträchtlichen Aufwand erfordern.

Der Erfindung liegt die Aufgabe zugrunde, einen Schraubendreher zu schaffen, der die vorgenannten Nachteile vermeidet und eine sichere Handhabung durch den Chirurgen gestattet.

Erfindungsgemäß wird die Aufgabe durch einen Schraubendreher mit den Merkmalen aus Anspruch 1 gelöst. Vorteilhafte Ausgestaltungen bilden die Gegenstände der Unteransprüche.

Der erfindungsgemäße Schraubendreher ist für Knochenschrauben, insbesondere für Kompressions- und Verriegelungsschrauben an Knochennägeln vorgesehen. Der Schraubendreher besitzt einen vorzugsweise länglichen Handgriff, mit einem zentral aus diesem vorstehenden Schaft, an dessen freiem Ende ein Schraubendrehwerkzeug vorgesehen ist. Der Schaft weist einen flexiblen Schaftabschnitt auf. Erfindungsgemäß besitzt der Schaft zwei starre, massive Abschnitte, die an beiden Seiten des flexiblen Schaftabschnitts vorgesehen sind. Der flexible Schaftabschnitt besitzt bevorzugt an seinen Enden jeweils einen Halteabschnitt und ein zwischen diesen gehaltenes flexibles Element. Bevorzugt besteht das flexible Element aus einem flexiblen Rohrelement oder aus einem wendelförmig gewickelten Draht. Mindestens der flexible Schaftabschnitt ist von einem elastischen Schutzschlauch umgeben. Die Anordnung des flexiblen Schaftabschnitts zwischen zwei starren Schaftabschnitten erlaubt eine sichere und präzise Handhabung, bei der der Chirurg eine gute Kontrolle über das ausgeübte Drehmoment besitzt. Der flexible Schaftabschnitt mit einem flexiblen Element schafft die Möglichkeit zu einer stufenlosen Biegung des Schafts. Der mindestens über dem elastischen Schaftabschnitt angeordnete elastische Schutzschlauch verhindert das Eindringen von Blut und Gewebe in den flexiblen Schaftabschnitt und erleichtert so Reinigung und Desinfektion des Schraubendrehers nach dem Gebrauch.

In der Ausgestaltung des flexiblen Elements als ein flexibles Rohrelement ist dieses bevorzugt durch ein Metallrohr gebildet, dessen Außenwand Schlitze aufweist, die sich quer zur Längsrichtung erstrecken. Der Schutzschlauch besteht bevorzugt aus einem biokompatiblen Material, insbesondere eignet sich Silikon.

In einer bevorzugten Ausgestaltung ist der Durchmesser des Schafts in seinem flexiblen Abschnitt größer als in den starren Schaftabschnitten.

Der Querschnitt des Schafts zwischen Handgriff und flexiblen Abschnitt vergrößert sich hin zum flexiblen Abschnitt kegelförmig. Auch kann sich der Querschnitt des Schafts zwischen Werkzeug und flexiblem Bereich zum Werkzeug hin kegelförmig verjüngen.

In einer bevorzugten Ausgestaltung besitzt der Handgriff mit einem im wesentlichen runden Querschnitt eine abgeflachte Seite, wobei die abgeflachte Seite die Orientierung des Schraubendreherwerkzeugs anzeigt. Hierdurch kann der den Schraubendreher handhabende Operateur, auch wenn die Werkzeugspitze nicht oder nur schlecht sichtbar ist, sich einen Eindruck von dessen Orientierung verschaffen.

Zur besseren Handhabung des Schraubenziehers hat es sich als besonders vorteilhaft herausgestellt, daß der flexible Abschnitt eine Länge besitzt, die weniger als die halbe Gesamtlänge beträgt. Bevorzugt ist die Länge des flexiblen Abschnitts jedoch bezogen auf die Gesamtlänge länger als ein Drittel der Gesamtlänge.

Bei dieser Ausgestaltung ist der starre Schaftabschnitt mit dem Schraubendreherwerkzeug kürzer als der zweite starre Schaftabschnitt.

In einer bevorzugten Ausgestaltung ist das Schraubendrehwerkzeug einstückig an dem freien Ende des starren Schaftabschnitts geformt. In einer zweckmäßigen Ausgestaltung besitzen die Halteabschnitte und das flexible Element einen Durchmesser von ungefähr 10 cm, wobei bevorzugt der Schlauch eine Wandstärke von ungefähr 2 mm besitzt.

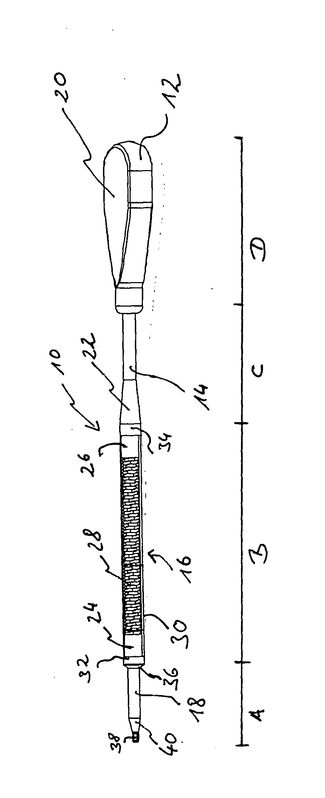

Eine bevorzugte Ausführungsform des erfindungsgemäßen Schraubendrehers wird nachfolgend anhand der Figur näher erläutert.

Die einzige Figur zeigt einen medizinischen Schraubendreher 10, mit einem Handgriff 12, einem starren Schaftabschnitt 14, einem flexiblen Schaftabschnitt 16 und einem zweiten starren Schaftabschnitt 18. Der Handgriff 12 besitzt eine abgeflachte Seite 20, die sich entlang der Schaftlängsachse erstreckt. Aus dem Handgriff 12 steht der starre und massiv ausgeführte Schaftabschnitt 14 vor, dessen Durchmesser sich im Übergang zum flexiblen Abschnitt 16 in einem Bereich 22 kegelförmig erweitert.

Der flexible Abschnitt 16 besitzt an seinen Enden jeweils einen Halteabschnitt 22 und 24, zwischen denen ein wendelförmig gewickelter Draht, beispielsweise aus Federstahl, gehalten ist. Auf den Abschnitten 24 und 26 sowie über den flexiblen Draht ist ein Schutzschlauch 30 aus elastischem, biokompatiblem Material gezogen. In dem dargestellten Ausführungsbeispiel handelt es sich bei dem Schutzschlauch um einen Schlauch aus Silikonmaterial mit einer Wandstärke von ungefähr 2 mm.

Neben der dargestellten Variante, bei der der flexible Bereich 28 aus einem wendelförmig gewickelten Draht hergestellt ist, ist es auch möglich, in den flexiblen Bereich mit einem dünnwandigen Metallrohr zu bilden, dessen Außenwand durch quer zur Längsachse stehende Schlitze durchbrochen ist, wodurch eine Biegung des Metallrohrs möglich wird. Alternativ können auch profilierte dünnwandige Metallrohre eingesetzt werden, um den flexiblen Abschnitt 16 zu schaffen. Wichtig bei der Verwendung eines Metallrohrs ist, daß innerhalb eines vorbestimmten Winkelbereichs eine nahezu stufenlose Biegung erfolgen kann.

Der Schutzschlauch 30 schließt an seinen beiden Enden bündig mit der Außenwand 32 und 34 an dem Schaft ab.

Der starre Abschnitt 18 schließt mit einem kegelförmigen Abschnitt 36 an den flexiblen Bereich 16 an. Die Werkzeugspitze 38 ist an dem starren Bereich 18 geformt und geht über einen sich ebenfalls kegelförmig erweiternden Bereich 40 in den starren Bereich 18 über.

Die Gesamtlänge des erfindungsgemäßen Schraubendrehers setzt sich zusammen aus der Länge A des starren Abschnitts 18, der Länge B des flexiblen Abschnitts 16, der Länge C des starren Abschnitts 14 und der Länge D des Handgriffs 12. Die Längenverhältnisse sind hierbei derart gewählt, daß der starre Abschnitt 18 mit seiner Länge A kürzer als der starre Abschnitt 14 mit seiner Länge C ist (A<C). Der flexible Abschnitt 16 mit seiner Länge B ist länger als jeder der starren Abschnitte und der Handriff (B>A, B>C, B>D). Bezogen auf die Gesamtlänge (A+B+C+D) besitzt der flexible Abschnitt 16 eine Länge B, die kleiner als die halbe Gesamtlänge (2B<A+B+C+D) und größer als ein Drittel der Gesamtlänge (3B>A+B+C+D) ist.

Der dargestellte Schraubendreher mit flexiblem Schaft ist besonders zum Antrieb von Schrauben geeignet, die in einem Knochennagel angeordnet sind, wie beispielsweise Kompressions- oder Verriegelungsschrauben. Der besondere Vorteil liegt darin, daß der Antrieb der Schraube mit der Hand nicht in gradliniger Verlängerung der Schraubenachse ausgeführt werden muß. So wird vermieden, daß der Raum für den Schraubendrehergriff durch den Körper des Patienten blockiert ist, wie es beispielsweise durch die Beckenschaufel beim anterograden Zugang im Femur erfolgt.

## Patentansprüche

1. Schraubendreher für Knochenschrauben, insbesondere für Kompressions- und Verriegelungsschrauben, mit
- einem Handgriff (12),
- einem zentral aus dem Handgriff vorstehenden Schaft (14, 16, 18), der an seinem Ende ein Schraubendrehwerkzeug (38) aufweist,
- wobei der Schaft einen flexiblen Schaftabschnitt (16) besitzt,
**dadurch gekennzeichnet, daß**
- der Schaft zwei starre, massive Abschnitte (14, 18) auf beiden Seiten des flexiblen Abschnitts (16) besitzt,
- der flexible Schaftabschnitt (16) ein flexibles Element (28) zur Übertragung eines Drehmoments aufweist und
- mindestens der flexible Schaftabschnitt (16) von einem elastischen Schutzschlauch (30) umgeben ist.

2. Schraubendreher nach Anspruch 1, **dadurch gekennzeichnet, daß** der flexible Schaftabschnitt an seinen Enden jeweils einen Halteabschnitt (24, 26) aufweist, wobei zwischen den Halteabschnitten das flexible Element gehalten ist.

3. Schraubendreher nach Anspruch 1 oder 2, **dadurch gekennzeichnet, daß** das flexible Element (28) aus einem wendelförmig gewickelten Draht besteht.

4. Schraubendreher nach Anspruch 1 oder 2, **dadurch gekennzeichnet, daß** das flexible Element (28) aus einem flexiblen Rohrelement besteht.

5. Schraubendreher nach Anspruch 4, **dadurch gekennzeichnet, daß** das flexible Rohrelement mit quer zur Längsrichtung verlaufenden Schlitzen versehen ist.

6. Schraubendreher nach Anspruch 1 bis 5, **dadurch gekennzeichnet, daß** der Schutzschlauch aus einem biokompatiblen Material, insbesondere aus Silikon besteht.

7. Schraubendreher nach einem der Ansprüche 1 bis 6, **dadurch gekennzeichnet, daß** der Durchmesser des Schafts im flexiblen Abschnitt (16) größer als in den starren Abschnitten (14, 18) ist.

8. Schraubendreher nach einem der Ansprüche 1 bis 7, **dadurch gekennzeichnet, daß** der Querschnitt des Schafts zwischen Handgriff und flexiblem Bereich (16) sich zum flexiblen Bereich kegelförmig (22) hin vergrößert.

9. Schraubendreher nach einem der vorangegangenen Ansprüche, **dadurch gekennzeichnet, daß** der Querschnitt des Schafts zwischen Werkzeug (38) und flexiblem Bereich (16) sich zum Werkzeug hin kegelförmig (36, 40) verjüngt.

10. Schraubendreher nach einem der vorangegangenen Ansprüche, **dadurch gekennzeichnet, daß** der Handgriff (12) einem im wesentlichen runden Querschnitt mit einer abgeflachten Seite (20) aufweist, wobei die abgeflachte Seite (20) die Orientierung des Schraubendrehwerkzeugs (38) anzeigt.

11. Schraubendreher nach einem der vorangegangenen Ansprüche, **dadurch gekennzeichnet, daß** der flexible Abschnitt (16) eine Länge (B) aufweist, die weniger als die halbe Gesamtlänge (A+B+C+D) beträgt.

12. Schraubendreher nach einem der vorangegangenen Ansprüche, **dadurch gekennzeichnet, daß** der flexible Abschnitt (16) eine Länge (B) aufweist, die mehr als ein Drittel der Gesamtlänge (A+B+C+C) beträgt.

13. Schraubendreher nach einem der vorangegangenen Ansprüche, **dadurch gekennzeichnet, daß** der starre Schaftabschnitt (18) mit dem Schraubendrehwerkzeug (38) kürzer als der zweite starre Schaftabschnitt (14) ist.

14. Schraubendreher nach einem der vorangegangenen Ansprüche, **dadurch gekennzeichnet, daß** das Schraubendrehwerkzeug (38) einstückig an einem freien Ende des starren Schaftabschnitts (18) geformt ist.

15. Schraubendreher nach einem der vorangegangenen Ansprüche, **dadurch gekennzeichnet, daß** die Halteabschnitte (24, 26) und das elastische Element einen Durchmesser von ungefähr 10 mm besitzen.

16. Schraubendreher nach einem der vorangegangenen Ansprüche, **dadurch gekennzeichnet, daß** der Schlauch (30) eine Wandstärke von ungefähr 2 mm besitzt.
